# FASCICULE DE BREVET EUROPEEN

(11) **EP 1 655 281 B1**
(45) Date de publication et mention de la délivrance du brevet: **23.12.2009**
(21) Numéro de dépôt: 04105467.7
(22) Date de dépôt: 03.11.2004
(51) Int. Cl.: C07C 233/47, C07C 231/02, C07C 231/12, A61K 31/221, A61K 31/30, A61K 31/28, A61P 29/00, A61P 31/02, A61P 31/04, A61P 39/06

(54) **Sels de N-acyl-L-aspartate-b-mono-ester et/ou de N-acyl-L-glutamate-g-mono-ester, leur procédé de préparation et leur utilisation dans des compositions thérapeutique ou cosmétique**
Salze von N-Acyl-L-aspartate-beta-monoestern und/oder N-Acyl-L-glutamate-gamma-monoestern, Verfahren zu ihrer Herstellung sowie ihre Verwendung in therapeutischen oder kosmetischen Zusammensetzungen
Salts of N-acyl-L-aspartate-beta-mono-ester and/or of N-acyl-L-glutamate-gamma-monoester, the process for preparing them and their use in therapeutic or cosmetic compositions

(43) Date de publication de la demande: 10.05.2006
(73) Titulaire: Laboratoires Asepta, Immeuble La Ruche (MC)
(72) Inventeur: Dencausse, Laurent, 06000, Nice (FR); Clamou, Jean-Luc, 06000, Nice (FR); Lacroix, M. Georges, 06000, Nice (FR); Artaud, M. Jacques, 06000, Nice (FR)
(74) Mandataire: Decobert, Jean-Pascal

(56) Documents cités:
- EP-A- 0 928 608
- EP-A- 1 072 251
- FR-A- 2 705 341

## Description

La présente invention concerne des sels de N-acyl-L-aspartate-β-mono-ester et/ou de N-acyl-L-glutamate-γ-mono-ester.

L'invention vise également le procédé de préparation de ces sels ainsi que leur utilisation notamment dans des compositions pharmaceutiques, cosmétiques ou dermatologiques.

Les sels métalliques et les oligo-éléments interviennent à de nombreux niveaux de la physiologie cutanée et tiennent une place importante en thérapeutique dermatologique. Toute carence se traduit par une déshydratation et un vieillissement prématuré de l'épiderme, avec une altération du tonus et de l'élasticité.

Les sels métalliques et les oligo-éléments constituent donc une source d'actifs particulièrement intéressants pour l'industrie des cosmétiques (Cosmétologie, n°8, Octobre 1995 - p 44-47).

Les sels de zinc (Zn), connus pour leur activité anti-inflammatoire et pour leurs propriétés sur la prolifération et la différentiation kératinocytaire, sont utilisés depuis longtemps dans le traitement de l'acné et autres lésions cutanées. La repousse des cheveux constitue également une cible d'action potentielle de ces sels qui permettent d'avoir une réponse thérapeutique dans le cas de pelade ou de dystrophie pilaire (Les nouvelles Dermatologiques, volume 16 - n°10 - 1997 - p 432-433). Une carence en zinc entraîne des ulcérations de la peau, une chute des cheveux et diverses manifestations dermatologiques plus ou moins graves (retard de la cicatrisation, maladies graves chez le nourrisson). Le zinc aide à la cicatrisation des lésions cutanées, il est indispensable aux actions de nombreuses enzymes et entre dans la régulation de la glycémie (diabète) car il est naturellement présent dans la composition de l'insuline.

Les propriétés anti-infectieuses des sels de cuivre (Cu) et leurs capacités à induire la production de collagène sont également exploitées avec beaucoup d'intérêt. Le cuivre joue également un rôle dans le maintien du pH physiologique cutané et contribue à l'élaboration de la mélanine, pigment responsable de la coloration de la peau. Le cuivre catalyse également les réactions chimiques liées à l'oxygène, il constitue un élément trace dont la concentration dans le tissu conjonctif dermique est extrêmement faible, mais dont la présence est indispensable à toutes les réactions d'oxydo-réduction dans les cellules du derme. (Les nouvelles esthétiques, janvier 1991, p 34).

Les sels de sélénium (Se) qui participent à la stabilisation des molécules de kératine en s'incorporant au niveau des ponts disulfures sont considérés comme des agents antiradicalaires et sont de ce fait utilisés dans la prévention du photo vieillissement cutané. Le sélénium est un constituant de la glutathion-péroxydase, enzyme qui joue un rôle d'antioxydant intracellulaire, identique à celui de la vitamine E. Cet effet antioxydant est capital dans la détoxication des radicaux libres produits par le métabolisme cellulaire.

Le cobalt (Co) est un composant de la vitamine B12 (la cobalamine), qui est essentielle dans la croissance des cellules, d'autre part, le cobalt possède une action bénéfique sur les problèmes circulatoires.

Les sels de manganèse (Mn) qui favorisent la production de collagène sont utilisés en association avec les sels de cuivre dans les traitements des troubles de la cicatrisation (Les nouvelles Dermatologiques, volume 16 - n°8 - 1997 - p 374-377). Le manganèse joue également un rôle important au niveau du système immunitaire de la peau. Dans les cas de dermatoses, une carence en manganèse a été souvent observée.

Les sels de fer (Fe) ont également une implication importante dans la maturation de la peau, des ongles et des cheveux car ils constituent un nutriment essentiel du métabolisme de l'oxygène et de la fonction mitochondriale. Le fer est de ce fait un élément indispensable à l'oxygénation des tissus (International journal of Cosmetic Science, 2001, 23, p 129-137).

Actuellement, les sels métalliques ou minéraux sont employés couramment dans l'industrie cosmétique sous des formes très variées. On trouve des composés naturels d'origine minérale tels que les oxydes de zinc (ZnO₂), de silicium (SiO₂), de titane (TiO₂), de fer (FeO₂), de manganèse (MnO₂) et d'aluminium (AlO₂). Cependant, la bio disponibilité de ces sels n'est pas toujours optimale, car ils peuvent présenter une solubilité réduite dans l'eau et certaines huiles, limitant ainsi leurs applications galéniques (Cahiers d'Esthétique -Cosmétique, MC Martini et al., 1995, ed. Simep/Masson).

D'autre part, certains de ces sels présentent des effets secondaires gênants en cosmétologie (propriétés astringentes, produits parfois trop agressifs...).

À coté de ces produits naturels, de très nombreux sels minéraux organiques ont été développés par voie chimique afin de faciliter et d'améliorer leurs utilisations cosmétiques et thérapeutiques.

Ainsi, de nombreux sels métalliques et minéraux organiques ont été synthétisés, plus particulièrement à partir d'acides gras, d'acides aminés, d'hydrolysats de protéines et également de N-acylamino acides (association d'acides gras et d'acides aminés). Une liste non exhaustive des produits enregistrés auprès de " *l'International Cosmetic Ingredient Dictionary and Handbook"* édition 2002 est donnée ci-dessous à titre d'exemple.

### Sels réalisés à partir des acides gras :

- Caprylate d'aluminium CAS n° [6028-57-5]
- Distéarate d'aluminium CAS n° [300-92-5]
- Isostéarate d'aluminium CAS n° [72277-75-9]
- Myristate d'aluminium CAS n° [4040-50-0]
- Stéarate d'aluminium CAS n° [7047-84-9]
- Laurate de zinc CAS n° [2452-01-9]
- Myristate de zinc CAS n° [16260-27-8]
- Palmitate de zinc CAS n° [4991-47-3]
- Ricinoléate de zinc CAS n° [13040-19-2]
- Stéarate de zinc CAS n° [557-05-1]
- Undecylenate de zinc CAS n° [557-08-4]
- Myristate de magnésium CAS n° [4086-70-8]
- Palmitate de magnésium CAS n° [2601-98-1]
- Propionate de magnésium CAS n° [557-27-7]
- Stéarate de magnésium CAS n° [557-04-0]
- Laurate de potassium CAS n° [10124-65-9]
- Linoléate de potassium CAS n° [3414-89-9]
- Myristate de potassium CAS n° [13429-27-1]
- Palmitate de potassium CAS n° [2624-31-9]
- Ricinoléate de potassium CAS n° [7492-30-0]
- Stéarate de potassium CAS n° [593-29-3]
- Oléate de potassium CAS n° [143-18-0]
- Undécylénate de potassium CAS n° [6159-41-7]
- Laurate de calcium CAS n° [4696-56-4]
- Myristate de calcium CAS n° [15284-51-2]
- Propionate de calcium CAS n° [4075-81-4]
- Stéarate de calcium CAS n° [1592-23-0]
- Undécylénate de calcium CAS n° [1322-14-1]
- Behenate de calcium CAS n° [3578-72-1]
- Acétate de cuivre CAS n° [142-71-2]

### Sels réalisés à partir d'acides aminés et d'hydrolysats de protéines :

- Aspartate de calcium CAS n° [21059-46-1]
- Aspartate de manganèse
- Aspartate de sélénium
- Aspartate de magnésium CAS n° [2068-80-6]
- Aspartate de zinc CAS n° [36393-20-1]
- Cystéinate de zinc
- Glutamate de zinc CAS n° [34992-53-5]
- Glycinate de zinc CAS n° [14281-83-58]
- Potassium caséinate CAS n° [68131-54-4]
- Aluminium glutamate de suif hydrogéné
- Potassium de suif CAS n° [61790-32-7]
- Magnésium de suif
- Zinc collagénate

### Sels réalisés à partir de N-acylaminoacides :

- Zinc undecenoyl acides aminés de blé
- Aluminium capryloyl acides aminés de collagène
- Aluminium undecenoyl acides aminés de collagène
- Capryloyl glutamate de potassium
- Cocoyl glutamate de potassium
- Potassium cocoyl amino acides de caséine, de levures, de maïs, de kératine, de soie, de soja, d'avoine, de riz, de blé et de collagène CAS n° [68920-65-0]
- Lauroyl glutamate de potassium CAS n° [89187-78-0]
- Myristoyl glutamate de potassium
- Potassium lauroyl amino acides de blé, de soja, de collagène
- Potassium myristoyl amino acides de collagène
- Potassium oléoyl amino acides de collagène
- Potassium palmitoyl amino acides de maïs, de blé et d'avoine
- Potassium undecenoyl amino acides de maïs, de blé, de soja et de collagène CAS n° [68951-92-8]
- Undecenoyl glutamate de potassium
- Palmitoyl glutamate de magnésium CAS n° [57539-47-6]

D'une manière générale, de nombreux brevets ont été publiés concernant la préparation et l'utilisation de sels minéraux et de sels métalliques obtenus à partir d'acides gras, d'acides aminés et d'hydrolysats de protéines.

Ainsi, le FR 9512656 décrit l'utilisation de sels (lanthanide, étain, zinc, manganèse, yttrium, cobalt, baryum et strontium) pour le traitement des désordres cutanés (zona, brûlures, eczematides, ulcères cutanés, cicatrisations difficiles). Les sels cités sont des carbonates, des bicarbonates, des sulfates, des glycérophosphates, des borates, des chlorures, des nitrates, des acétates, des hydroxydes, des persulfates, des sels α-hydroxy-acides, des sels d'acides aminés (dont l'aspartate) et des sels d'acides gras (palmitate, oléate, caséinate, béhénate).

Le GB 2297975 fait état d'une invention concernant une composition nettoyante comprenant des sels d'acides gras pris individuellement ou en mélange. Les sels décrits sont le zinc, le calcium, le magnésium, le lithium et l'aluminium. Les acides gras utilisés ont une condensation en carbone de 14 à 22 atomes.

Le WO 98/17269 décrit une utilisation de sels de zinc de l'acide linoléique. La composition est destinée au traitement cutané d'eczéma, de psoriasis et de dermites.

L' EP 0048 473 fait état d'une composition pharmaceutique renfermant des sels de zinc. Ces sels sont choisis parmi les sulfates, carbonates et phosphates et associés avec des acides aminés tels que l'acide L-glutamique, glycine, L-leucine, L-arginine, L-thréonine et L-proline. La composition est destinée à prévenir les insuffisances en zinc selon un mode d'administration par voie orale.

Le FR 7513659 décrit également des associations de sels de zinc (chlorures, nitrates, sulfates, carbonates et acétates) et d'acides aminés comprenant l'acide aspartique et l'acide glutamique. La composition est destinée à l'oligothérapie par administration par voie orale.

L'EP 0415 598 présente l'utilisation de N-acylamino acides et de leurs sels pour réaliser une composition capillaire pour la repousse des cheveux. Les N-acylamino acides décrits incluent des dérivés de l'acide aspartique et de l'acide glutamique pris sous leur forme di-acide ou de sels de sodium (Na⁺), de potassium (K⁺) et de sels de triéthanolamine.

De nombreux documents font également état de N-acylamino acides réalisés à partir d'hydrolysats de protéines animales ou végétales (FR 2503144, FR 2503151, FR 2676741, EP 0601911, WO 92/20647, WO 94/26694). Les N-acylamino acides (ou lipoaminoacides) obtenus sont généralement pris sous leur forme acide libre ou sous leur forme salifiée suivant le type de composition recherchée. Les agents de salification sont choisis parmi l'ammoniaque, les cations de métaux alcalins (Na, K), les alcalino-terreux (Ca, Mg), les cations de métaux (Co, Fe, Mn, Cu, Zn, Al) et les bases organiques telles les mono, di ou triéthanolamine, la lysine, l'arginine, l'histidine, l'ornithine, la choline ou la morpholine.

A la lecture de ces différents documents, il apparaît que les sels métalliques (ou minéraux) bivalents, trivalents, tétravalents, pentavalents, hexavalents ou heptavalents de N-acyl-L-aspartate-β-mono-ester et de N-acyl-L-glutamate-γ-mono-ester objets de l'invention ne sont pas décrits.

A notre connaissance, seul le brevet EP 0572167 mentionne la réalisation de dérivés de l'acide L-glutamique et de ses sels pour le traitement et la repousse des cheveux. Les produits décrits incluent les esters en α et les esters en γ du N-acyl-L-glutamate de sodium, de potassium, de lithium et d'ammonium correspondants. À l'exception des sels d'ammonium quaternaires qui sont des sels organiques, les sels décrits sont des sels alcalins monovalents et ne comprennent pas les cations bivalents, trivalents, tétravalents, pentavalents, hexavalents ou heptavalents provenant des alcalino-terreux, des métaux de transition, des métalloïdes, des lanthanides et actinides. D'autre part, les sources comprenant l'acide L-aspartique et les hydrolysats de protéines ne sont pas abordés dans le brevet précité.

Ainsi, l'invention a pour premier objet des sels de N-acyl-L-aspartate-β-mono-ester et/ou de N-acyl-L-glutamate-γ-mono-ester ayant la formule générale suivante : 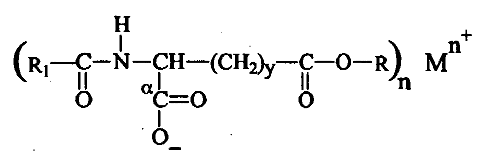 dans laquelle Mⁿ⁺ représente un métal ou un oligo-élément caractérisé par un état de valence n supérieur à l'unité (2 ≤ n ≤ 7), R₁ représente un radical alkyle, aryle, saturé ou insaturé de condensation ayant de 1 à 30 atomes de carbone, R est choisi parmi la partie hydrocarbone d'alcools linéaires ou ramifiés, cycliques ou acycliques et y est égal à 1 pour l'acide aspartique et est égal à 2 pour l'acide glutamique.

L'invention a pour deuxième objet un procédé de préparation des sels ayant la formule ci-dessus, comprenant les étapes suivantes :
- l'estérification par un alcool linéaire ou ramifié du ou des acides aminés di-acides L-aspartique et L-glutamique et éventuellement des acides aminés mono-acides associés provenant des hydrolysats de protéines ;
- l'hydrolyse régiosélective des fonctions esters situées en α des di-esters des acides aminés L-aspartique et L-glutamique présents dans le mélange ;
- la N-acylation en milieu organique de tous les esters d'acides aminés présents dans le mélange par un halogénure d'acide alkyle, aryle saturé ou non ;
- la salification par des bases mono-sodiques ou mono-potassiques de la fonction acide carboxylique libre située en α des dérivés N-acyl-L-aspartique-β-mono-ester et des dérivés N-acyl-L-glutamique-γ-mono-ester de façon à obtenir les sels mono-sodiques ou mono-potassiques correspondants ;
   ce procédé étant caractérisé par l'étape suivante :
- la substitution des sels alcalins mono-sodiques ou mono-potassiques des N-acyl-L-aspartate-β-mono-ester et N-acyl-L-glutamate-γ-mono-ester avec des sels métalliques bivalents, trivalents, tétravalents, pentavalents, hexavalents ou heptavalents provenant de métaux, d'alcalino-terreux, de métaux de transition incluant les lanthanides et les actinides.

Les métaux complexants Mⁿ⁺ des sels selon l'invention ou obtenus par le procédé selon l'invention sont de préférence choisis :
- soit parmi les cations bivalents (n=2) suivants : le zinc, le cuivre, le nickel, le manganèse, le magnésium, le calcium, le cobalt, le vanadium, le titane, le chrome, le fer, le silicium, et le cadmium,
- soit parmi les cations trivalents (n=3) suivants : l'aluminium, le rhodium, le gallium et l'or,
- soit parmi les cations tétravalents (n=4) suivants : le sélénium, le platine et le palladium,
- soit parmi les cations pentavalents (n=5) suivants : le tantale et le bismuth,
- soit parmi les cations hexavalents (n=6) comme le molybdène ou parmi les cations heptavalents (n=7) comme le rhénium.

Pour la mise en oeuvre du procédé de préparation des sels selon l'invention, de préférence :
- les acides aminés di-acides L-aspartique et L-glutamique sont pris individuellement ou en mélange ou bien en combinaison avec des hydrolysats de protéines animales ou végétales,
- les hydrolysats de protéines sont choisis de préférence à partir des protéines suivantes : la fibroïne de soie du *Bombyx mori,* le blé, le maïs, le soja, le tournesol, le lupin, la caséine et les algues marines *Laminaria digitata, Ascophyllum nodosum, Fucus vesiculosus, Chondrus crispus, Lithothamnium calcareum, Alsidium helminthocorton* et *Digenea simplex,*
- les hydrolysats de protéines animales ou végétales peuvent être préalablement enrichis en acides aminés di-acides L-aspartique et/ou L-glutamique dans des proportions massiques pouvant aller respectivement de 5 à 95 % par rapport aux hydrolysats de protéines,
- l'hydrolyse des fonctions esters situées en α des di-esters des acides aminés L-aspartique et L-glutamique est régiosélective,
- les sels réalisés sont des sels bivalents de zinc di-(N-palmitoyl-L-glutamate-γ-methyl-ester) et des sels bivalents de zinc di-(N-palmitoyl-L-aspartate-β-methyl-ester) ou des sels bivalents de cuivre di-(N-palmitoyl-L-glutamate-γ-methyl-ester) et des sels bivalents de cuivre di-(N-palmitoyl-L-aspartate-β-methyl-ester), ou des sels bivalents de zinc di-(N-palmitoyl- L-glutamate-γ-methyl-ester) de fibroïne de soie, ou des sels trivalents d'aluminium tri-(N-palmitoyl-L-glutamate-γ-methyl-ester) de caséine du lait de vache.

Selon un troisième objet de l'invention, les sels selon l'invention ou obtenus par le procédé selon l'invention sont utilisés dans des compositions pharmaceutiques, cosmétiques ou dermatologiques et de préférence pour l'obtention de molécules ayant une activité anti-inflammatoire, ou pour l'obtention de molécules ayant une activité anti-microbienne, ou pour l'obtention d'agents anti-sudoraux, ou pour l'obtention de molécules ayant une activité sur la régénération cutanée cellulaire, ou pour l'obtention d'agents anti-radicalaires.

On va maintenant décrire plus en détail le procédé de préparation de sels métalliques ou de sels minéraux bivalents, trivalents, tétravalents, pentavalents, hexavalents ou heptavalents de N-acyl-L-aspartate-β-mono-ester et de N-acyl-L-glutamate-γ-mono-ester. Le protocole de synthèse permettant l'obtention de ces sels comprend les cinq réactions suivantes :

### 1^{ère} étape

L'estérification totale de l'acide L-aspartique (y = 1) et/ou de l'acide L-glutamique (y = 2) par un alcool ROH afin d'obtenir les diesters correspondants :

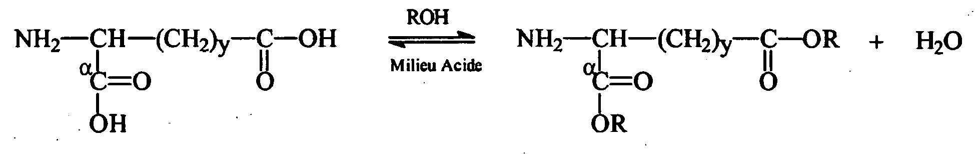

### 2^{e} étape

L'hydrolyse sélective par voie chimique ou enzymatique des fonctions esters situées en α des diesters des acides L-aspartique et L-glutamique en utilisant de préférence la carboxyl estérase de foie de porc (PLE) :

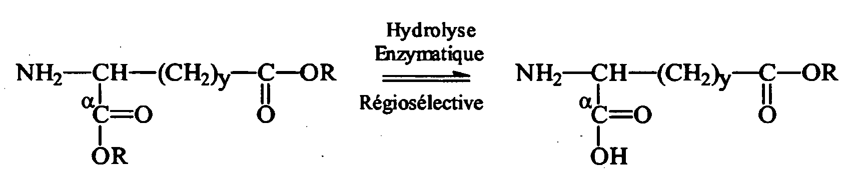

### 3^{e} étape

L'acylation de l'acide L-aspartique-β-mono-ester et/ou de l'acide L-glutamique-γ-mono-ester en présence d'un halogénure d'acide (alkyle, aryle saturé ou non), de préférence un chlorure d'acide (R₁COCl) :

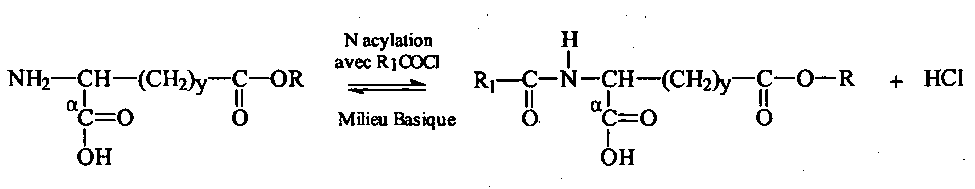

### 4^{e} étape

La salification contrôlée par une base BOH, de préférence la potasse (KOH) ou la soude (NaOH) de la fonction acide carboxylique libre située en α du dérivé N-acyl-L-aspartique-β-mono-ester et du dérivé N-acyl-L-glutamique-γ-mono-ester de façon à obtenir les sels mono-sodiques ou mono-potassiques correspondants :

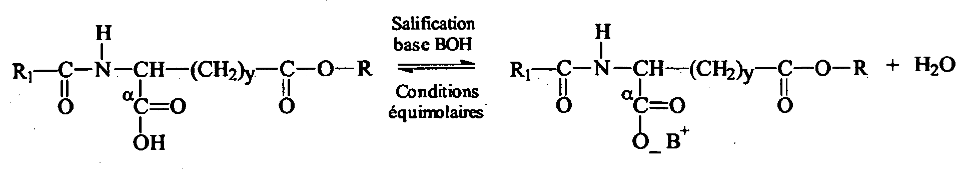

### 5^{e} étape

Les cations monovalents B⁺ des sels alcalins mono-sodiques ou mono-potassiques des N-acyl-L-aspartate-β-mono-ester et/ou N-acyl-L-glutamate-γ-mono-ester sont substitués par des sels métalliques ou minéraux Mⁿ⁺ bivalents, trivalents, tétravalents, pentavalents, hexavalents ou heptavalents selon les besoins, de préférence choisis parmi le zinc, le cuivre, le calcium et l'aluminium :

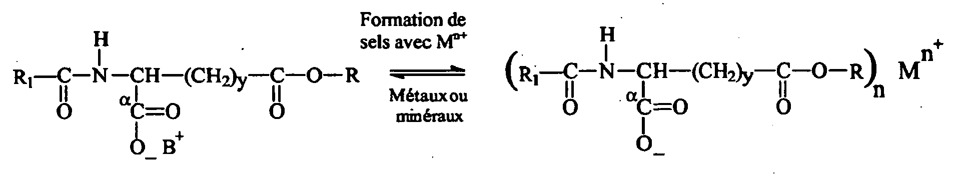

Il faut noter que dans certains cas l'étape 5 de formation des sels métalliques ou minéraux peut être réalisée directement à partir de l'étape 3.

Selon l'invention, on peut réaliser des sels métalliques ou minéraux bivalents, trivalents, tétravalents, pentavalents, hexavalents ou heptavalents de N-acyl-L-aspartate-β-mono-ester et/ou de N-acyl-L-glutamate-γ-mono-ester qui sont obtenus à partir d'hydrolysats de protéines animales ou végétales renfermant naturellement les acides L-aspartique et/ou L-glutamique. Selon les besoins, des protéines riches en acide L-aspartique (soja, tournesol, lupin, poisson) ou en acide L-glutamique (tournesol, blé, maïs, caséine) sont sélectionnées individuellement ou en mélange. Des hydrolysats de protéines marines (algues) renfermant les acides aminés di-acides sont également utilisés en particulier à partir de *Laminaria digitata, Ascophyllum nodosum, Fucus vesiculosus, Chondrus crispus, Lithothamnium calcareum, Alsidium helminthocorton* et *Digenea simplex.*

Selon les quantités initiales en acides L-aspartique et/ou L-glutamique, les hydrolysats de protéines pourront être préalablement enrichis dans des proportions massiques pouvant aller de 5% à 95%. L'enrichissement des hydrolysats de protéines en acides L-aspartique et/ou L-glutamique permet ainsi de moduler la quantité de sels formés dans le mélange final.

Le protocole de synthèse permettant l'obtention des sels de N-acyl-L-aspartate-β-mono-ester et de N-acyl-L-glutamate-γ-mono-ester réalisés à partir d'hydrolysats de protéines est identique à celui décrit précédemment, à l'exception de la source en acides aminés qui est constituée par un mélange d'acides aminés mono-acides et di-acides.

La première étape d'estérification conduit à l'obtention de mono-esters d'acides aminés (avec les mono-acides) et de di-esters d'acides aminés (avec les di-acides). La deuxième étape utilisant l'hydrolyse sélective par voie enzymatique affecte seulement les fonctions esters situées en α des diesters, à savoir les carbonyles en α des di-esters L-aspartique et L-glutamique. La troisième étape consacrée à l'acylation des mono-esters d'acides aminés et des acides L-aspartique-β-mono-ester et L-glutamique-γ-mono-ester est réalisée dans des conditions expérimentales identiques à celles décrites précédemment. Les quatrième et cinquième étapes de la synthèse sont destinées à transformer respectivement les N-acyl-L-aspartate-β-mono-ester et/ou les N-acyl-L-glutamate-γ-mono-ester en leur sels métalliques.

A l'issue du procédé de synthèse, seuls les di-acides présents naturellement dans les protéines ou additionnés artificiellement à savoir l'acide L-aspartique et l'acide L-glutamique conduisent à l'obtention des sels voulus, les autres acides aminés mono-acides présents dans les protéines étudiées permettent l'obtention de N-acylamino esters qui sont déjà décrits dans les brevets Asepta (EP 0882 702 et EP 1072 251). Cette synthèse permet l'association de N-acylamino esters pris en mélange avec des sels de N-acyl-L-aspartate-β-mono-ester et/ou des sels de N-acyl-L-glutamate-γ-mono-ester. Cela constitue une nouveauté très intéressante pour le contrôle des proportions d'ions métalliques dans le mélange final permettant ainsi de favoriser la bio disponibilité et la solubilité des sels dans les matrices.

D'une manière générale, les propriétés des sels obtenus sont fonctions de la nature du métal choisi, de l'acide aminé sélectionné parmi l'acide aspartique et/ou l'acide glutamique, de l'origine et de la composition des hydrolysats de protéines qui sont associés et également du taux d'enrichissement massique en acides aminés di-acides qui peut varier de 5 à 95%. Les propriétés intrinsèques des structures réalisées sont également dépendantes de la nature de la chaîne carbonée acylante et de l'alcool qui estérifie la position β de l'aspartate et de l'état d'estérification de la position γ du glutamate.

Ainsi, les chaînes acylantes renfermant quatorze (C14), seize (C16) et dix-huit atomes de carbone (C18) sont recherchées pour leur pouvoir restructurant au niveau de la peau, alors que les chaînes caprique (C6), caprylique (C8) et undécylénique (C11:1) sont préférées pour réaliser des structures antimicrobiennes (fongiques et bactéricides).

Les acides aspartique et glutamique sont généralement choisis pour leurs propriétés de régénération cellulaires car ils présentent un potentiel activateur favorable aux échanges ionotropiques. Ainsi, sous forme de sels : l'aspartate de magnésium, l'aspartate de calcium, le glutamate de sodium et le glutamate de calcium présentent des propriétés reconnues au niveau de la pénétration des membranes cellulaires.

Sous leur forme N-acylée, β ou γ estérifiée, le transport et la diffusion des sels d'aspartate et de glutamate dans la peau est améliorée, augmentant ainsi la compatibilité des sels formés avec les couches lipidiques de l'épiderme. D'une manière générale, les sels mono estérifiés obtenus présentent, de part leur fonction ester, une solubilité améliorée par rapport à leur homologues dibasiques.

L'excellente tolérance cutanée et l'innocuité des sels de N-acyl-L-aspartate-β-mono-ester et de N-acyl-L-glutamate-γ-mono-ester associées à leurs propriétés intrinsèques multiples (activité anti-inflammatoire, fongique, bactéricide, pouvoir cicatrisant et régénérateur...) selon les structures réalisées confèrent aux sels décrits des caractéristiques cosmétiques remarquables tels que l'onctuosité, la douceur, le pouvoir de dispersion et de pénétration qui sont autant de critères pour réaliser des formulations cosmétiques de qualité.

Ainsi, des sels possédant des propriétés cicatrisantes et régénérantes sont développés à partir de sels de cuivre du di-(N-palmitoyl-L-aspartate-β-methyl ester) et également des sels de manganèse du di-(N-palmitoyl-L-glutamate-γ-methyl ester). Les sels possédant des propriétés anti-inflammatoires sont préférentiellement obtenus à partir de sels de zinc du di-(N-palmitoyl-L-glutamate-γ-methyl ester).

Des sels de zinc et de cuivre du di-(N-undécénoyl-L-aspartate-β-methyl ester) et du di-(N-undécénoyl-L-glutamate-γ-methyl ester) sont synthétisés et utilisés pour leurs propriétés anti-inflammatoires et fongiques sur les micro organismes alors que les sels de zinc et de cuivre du di-(N-capryloyl-L-aspartate-β-methyl ester) et du di-(N-capryloyl-L-glutamate-γ-methyl ester) présentent des propriétés bactéricides et anti-inflammatoires.

Pour la prévention du photo vieillissement cutané et la recherche d'agents antiradicalaires, les sels de sélénium du tetra-(N-stéaroyl-L-glutamate-γ-methyl ester) et les sels de calcium du di-(N-stéaroyl-L-glutamate-γ-methyl ester) sont choisis.

Les différents sels cités précédemment peuvent être préférentiellement associés à des hydrolysats de protéines choisis de préférence parmi la soie (fibroïne et séricine), les hydrolysats de céréales (avoine, blé, maïs, riz) et les algues marines *(Laminaria digitata, Ascophyllum nodosum, Fucus vesiculosus, Chondrus crispus, Lithothamnium calcareum, Alsidium helminthocorton* et *Digenea simplex).*

Les exemples qui suivent décrivent en détail la préparation de certains sels de N-acyl-L-aspartate-β-mono-ester et de N-acyl-L-glutamate-γ-mono-ester obtenus à partir d'acide L-aspartique ou L-glutamique pris individuellement ou à partir de protéines naturelles préalablement enrichies.

### Exemple 1

### Synthèse de sels de zinc di-(N-palmitoyl-L-aspartate-β-methyl-ester)

### (1) Estérification de l'acide L-aspartique

Dans un ballon bicol de 250 ml sont pesés précisément 10 grammes d'acide L-aspartique (C₄H₇NO₄ - MM=133,1g - n° CAS [56-84-58]) soit 0,075 mole d'acide aminé. La masse est additionnée de 150 ml de méthanol puis de 2,2 équivalents de chlorure de thionyle (SOCl₂), soit 0,165 mole, soit une masse de 19,6 g et un volume de 12,0 ml. Le chlorure de thionyle est additionné goutte à goutte tout en maintenant le mélange méthanolique d'acide aminé à une température proche de 5°C (bain de glace). Lorsque la totalité du SOCl₂ a été additionnée, le mélange réactionnel est ensuite chauffé à reflux pendant 2 heures. Le méthanol et le SOCl₂ en excès sont éliminés à l'évaporateur rotatif sous vide. Le méthanol résiduel est épuisé par deux fois 100 ml d'hexane puis le mélange est évaporé sous vide à sec. Une fois recristallisés dans l'hexane, les esters méthyliques (diesters) de l'acide L-aspartique (MM = 197,5g) sont obtenus sous forme de chlorhydrate de couleur blanche. La masse obtenue est de 14,1 g, ce qui correspond à un rendement d'estérification de 95 %.

### (2) Hydrolyse enzymatique sélective de la position α du diester de l'acide L-aspartique

Les 14,1 g ou 0,071 mole de diester méthylique de l'acide L-aspartique sont repris dans un erlenmeyer de 500 ml et additionnés de 10 ml de méthanol puis de 100 ml de tampon phosphate à 0,01 M afin de maintenir le pH du mélange à la neutralité (pH = 7). Le mélange est agité à température ambiante puis additionné sous agitation de 4,7 g de PLE *(Porcine Liver Esterase* 15 U/mg, Sigma E 3019) soit environ 71000 unités. Le mélange est agité durant 5 heures. Le pH est maintenu à 7 par ajout d'une solution d'hydroxyde de sodium à 1M. Lorsque un équivalent de base est consommé, la réaction est stoppée avec de l'acide chlorhydrique (environ 1M) par acidification du milieu à pH 3-4. Le mélange est additionné de 50 ml de méthanol. L'acide L-aspartique-β-methyl-ester qui précipite est isolé par filtration puis séché à 40°C à l'étuve ventilée. La masse d'acide L-aspartique-β-methyl-ester obtenue est de 12,1 g, ce qui correspond à un rendement de réaction de 93 % soit 0,066 mole.

### (3) Condensation du chlorure de palmitoyle et de l'acide L-aspartique-β-methyl-ester

Les 12,1 g (0,066 mole) d'acide L-aspartique-β-methyl-ester sous forme de chlorhydrate sont placés dans un ballon bicol de 1 litre en présence de 200 ml de chlorure de méthylène et de 0,16 mole de triéthylamine soit 23 ml (2,5 équivalents / chlorhydrate d'acide L-aspartique-β-methyl-ester). Le mélange est refroidi entre 0-5°C puis additionné de 0,95 équivalent soit 0,062 mole ou 17,2 g de chlorure de palmitoyle en solution dans 30 ml de chlorure de méthylène. Après addition de l'agent acylant, le mélange est agité à 35°C pendant quatre heures puis transféré dans une ampoule à décanter. La phase organique est lavée successivement quatre fois par 250 ml d'eau acidulée pour éliminer la triéthylamine en excès. La phase organique est séchée sur sulfate de sodium, filtrée puis évaporée sous vide. La masse de N-palmitoyl-L-aspartate-β-methyl-ester obtenue est de 21,1 g, ce qui correspond à un rendement de condensation de 88 %.

### (4) Salification du N-palmitoyl-L-aspartate-β-methyl-ester

Les 21,1 g de N-palmitoyl-L-aspartate-β-methyl-ester soit 0,054 mole sont placés dans un erlenmayer de 500 ml et solubilisés dans 100 ml de méthanol. Le mélange est additionné d'une quantité équimolaire d'une solution aqueuse d'hydroxyde de sodium soit 0,054 mole de NaOH soit 2,23 g. Le mélange est agité durant deux heures à température ambiante, ce qui conduit à l'obtention du sel mono sodique du N-palmitoyl-L-aspartate-β-methyl-ester.

### (5) Substitution des sels mono sodiques du N-palmitoyl-L-aspartate- β-methyl-ester par le zinc

Les sels mono sodiques du N-palmitoyl-L-aspartate-β-methyl-ester sont substitués par le zinc par ajout d'une solution aqueuse de chlorure de zinc réalisée à partir de 0,5 équivalent de ZnCl₂ soit 0,027 mole et 3,7 g. Une fois la solution de chlorure de zinc additionnée, le sel de zinc di-(N-palmitoyl-L-aspartate-β-methyl-ester) précipite totalement dans le milieu. Le pH est amené à 3-4, puis le sel de zinc est filtré sur fritté sous vide, lavé à l'eau puis séché à l'étuve ventilée à 40°C jusqu'à l'obtention d'une poudre blanche. Le sel de zinc de formule brute (C₂₁H₃₈NO₅)₂ Zn (MM = 833,4g) est obtenu avec une masse de 21,5 g soit rendement massique de complexation de 95 %.

D'une manière générale, le rendement global de la synthèse est de 73 % (m/m) et les produits obtenus présentent un degré de pureté supérieur à 95 %.

### Exemple 2

Synthèse de sels de cuivre di-(N-palmitoyl-L-glutamate-γ-methyl-ester)

### (1) Estérification de l'acide L-glutamique

Dans un ballon bicol de 250 ml sont pesés précisément 10 grammes d'acide L-glutamique (C₅H₉NO₄ - MM=147,1g - n° CAS [56-86-00]) soit 0,067 mole d'acide aminé. La masse est additionnée de 150 ml de méthanol puis de 2,2 équivalents de chlorure de thionyle (SOCl₂), soit 0,149 mole, soit une masse de 17,7 g et un volume de 10,9 ml. Le chlorure de thionyle est additionné goutte à goutte tout en maintenant le mélange méthanolique d'acide aminé à une température proche de 5°C (bain de glace). Lorsque la totalité du SOCl₂ a été additionnée, le mélange réactionnel est ensuite chauffé à reflux pendant 2 heures. Le méthanol et le SOCl₂ en excès sont éliminés à l'évaporateur rotatif sous vide. Le méthanol résiduel est épuisé par deux fois 100 ml d'hexane puis le mélange est évaporé sous vide à sec. Une fois recristallisé dans l'hexane, le diester méthylique de l'acide L-glutamique (MM = 211,5g) est obtenu sous forme de chlorhydrate de couleur blanche. La masse obtenue est de 13,5 g, ce qui correspond à un rendement d'estérification de 95 %.

### (2) Hydrolyse enzymatique sélective de la position α du diester de l'acide L-glutamique

Les 13,5 g ou 0,063 mole de diester méthylique de l'acide L-glutamique sont repris dans un erlenmeyer de 500 ml et additionnés de 10 ml de méthanol puis de 100 ml de tampon phosphate à 0,01 M afin de maintenir le pH du mélange à la neutralité (pH = 7). Le mélange est agité à température ambiante puis additionné sous agitation de 4,2 g de PLE (*Porcine Liver Esterase* 15 U/mg, Sigma E 3019) soit environ 63000 unités. Le mélange est agité durant 5 heures. Le pH est maintenu à 7 par ajout d'une solution d'hydroxyde de sodium à 1M. Lorsque un équivalent de base est consommé, la réaction est stoppée avec de l'acide chlorhydrique (environ 1M) par acidification du milieu à pH 3-4. Le mélange est additionné de 50 ml de méthanol. L'acide L-glutamique-γ-methyl-ester qui précipite est isolé par filtration puis séché à 40°C à l'étuve ventilée. La masse d'acide L-glutamique-γ-methyl-ester obtenue est de 12,5 g, ce qui correspond à un rendement de réaction de 93 % soit 0,058 mole.

### (3) Condensation du chlorure de palmitoyle et de l'acide L-glutamique -γ-methyl-ester

Les 12,5 g (0,058 mole) d'acide L-glutamique-γ-methyl-ester sous forme de chlorhydrate sont placés dans un ballon bicol de 1 litre en présence de 200 ml de chlorure de méthylène et de 0,14 mole de triéthylamine soit 20 ml (2,5 équivalents / chlorhydrate d'acide L-glutamique-γ-methyl-ester). Le mélange est refroidi entre 0-5°C puis additionné de 0,95 équivalent soit 0,055 mole ou 15,1 g de chlorure de palmitoyle en solution dans 30 ml de chlorure de méthylène. Après addition de l'agent acylant, le mélange est agité à 35°C pendant quatre heures puis transféré dans une ampoule à décanter. La phase organique est lavée successivement quatre fois par 250 ml d'eau acidulée pour éliminer la triéthylamine en excès. La phase organique est séchée sur sulfate de sodium, filtrée puis évaporée sous vide. La masse de N-palmitoyl-L-glutamate-γ-methyl-ester obtenue est de 17,5 g, ce qui correspond à un rendement de condensation de 88 %.

### (4) Salification du N-palmitoyl-L-glutamate-γ-methyl-ester

Les 17,5 g de N-palmitoyl-L-glutamate-γ-methyl-ester soit 0,048 mole sont placés dans un erlenmeyer de 500 ml et solubilisés dans 100 ml de méthanol. Le mélange est additionné d'une quantité équimolaire d'une solution aqueuse d'hydroxyde de sodium soit 0,048 mole de NaOH soit 1,93 g. Le mélange est agité durant deux heures à température ambiante, ce qui conduit à l'obtention du sel mono sodique du N-palmitoyl-L-glutamate-γ-methyl-ester.

### (5) Substitution de sel mono sodique du N-palmitoyl-L-glutamate- γ-methyl-ester par le cuivre

Le sel mono sodique du N-palmitoyl-L-glutamate-γ-methyl-ester est substitué par le cuivre par ajout d'une solution aqueuse de chlorure de cuivre réalisée à partir de 0,5 équivalent de CuCl₂ soit 0,024 mole et 3,2 g. Une fois la solution de chlorure de cuivre additionnée, le sel de cuivre di-(N-palmitoyl-L-glutamate-γ-methyl-ester) précipite totalement dans le milieu. Le pH est amené à 3-4, puis le sel de cuivre est filtré sur fritté sous vide, lavé à l'eau puis séché à l'étuve ventilée à 40°C jusqu'à l'obtention d'une poudre colorée. Le sel de cuivre de formule brute (C₂₂H₄₀NO₅)₂ Cu (MM = 859,5g) est obtenu avec une masse de 19,6 g soit rendement massique de complexation de 95 %.

D'une manière générale, le rendement global de la synthèse est de 73 % (m/m) et les produits obtenus présentent un degré de pureté supérieur à 95 %.

### Exemple 3

### Synthèse de sels de zinc di-(N-palmitoyl-L-glutamate-γ-methyl-ester) d'hydrolysat de fibroïne de soie enrichi

### (1) Préparation des acides aminés de fibroïne de soie enrichi en acide L-glutamique

Dans un erlenmeyer, 10 g d'acides aminés de soie atomisés sont additionnés de 90 g d'acide L-glutamique selon les proportions massiques correspondants au mélange binaire 10 % d'acides aminés de soie atomisée et 90 % d'acide L-glutamique. La composition en acides aminés de la protéine de soie qui est utilisée est donnée dans le tableau 1.

**Tableau 1**

| Aminogramme des acides aminés atomisés de la soie (% m/m) | | | |
|---|---|---|---|
| Acides aminés | % massiques | Acides aminés | % massiques |
| Glycine | 45,3 | Tyrosine | 0,1 |
| Alanine | 42,9 | Isoleucine | traces |
| Sérine | 10,3 | Méthionine | traces |
| A. Glutamique | 0,5 | Phénylalanine | traces |
| Valine | 0,5 | Leucine | traces |
| A. Aspartique | 0,4 | Cystéine | traces |

Le pourcentage initial d'acide L-glutamique dans la soie utilisée est de 0,5 %. L'addition de 90 g d d'acide L-glutamique aux 10 g de substrat protéique permet d'augmenter artificiellement les proportions globales d'acide L-glutamique pour obtenir un pourcentage massique supérieur à 90 %. Cette addition d'acide L-glutamique permet de disposer d'un hydrolysat de soie particulièrement riche en acides aminés di-acides. La quantité d'acide aspartique présente initialement dans le mélange devient quasi négligeable par rapport à la quantité globale d'acide glutamique.

### (2) Estérification de l'hydrolysat de protéines de soie enrichi en acide L-glutamique

L'estérification de 100 g d'hydrolysat de soie enrichi acide L-glutamique est réalisée dans un ballon bicol de 500 ml. Les équivalences de réactifs à additionner sont calculées suivant le nombre total de moles d'acides aminés à estérifier. Le tableau 2 présente les pourcentages molaires de l'hydrolysat de soie atomisé enrichi en acide L-glutamique.

**Tableau 2**

| Pourcentages molaires des acides aminés de soie atomisés et enrichis en acide L-glutamique (10/90, m/m) | | | |
|---|---|---|---|
| Acides aminés | % molaires | Acides aminés | % molaires |
| Glycine | 0,06 | Tyrosine | 5,5.10⁻⁵ |
| Alanine | 0,05 | Isoleucine | traces |
| Sérine | 0,01 | Méthionine | traces |
| A. Glutamique (total) | 0,61 | Phénylalanine | traces |
| Valine | 4,0.10⁻⁴ | Leucine | traces |
| A. Aspartique | 3,0.10⁻⁴ | Cystéine | traces |

Sur les 100 g mis enjeux, le nombre total de moles à estérifier est égal à 0,73 (0,12 mole d'acides aminés mono-acides et 0,61 mole d'acide aminés di-acides, soit environ 84 %). Le mélange d'acides aminés est additionné de 350 ml de méthanol puis de 2,2 équivalents de SOCl₂ pour les di-acides, soit 1,3 moles et 1,2 équivalents de SOCl₂ pour les mono-acides, soit 0,14 mole ce qui ramène à une masse totale de SOCl₂ de 171,2 g et un volume de 105 ml. Le chlorure de thionyle est additionné goutte à goutte tout en maintenant le mélange méthanolique d'acides aminés à une température proche de 5°C (bain de glace). Lorsque la totalité du SOCl₂ a été additionnée, le mélange réactionnel est ensuite chauffé à reflux pendant deux heures. Le méthanol et le SOCl₂ en excès sont éliminés à l'évaporateur rotatif sous vide. Le méthanol résiduel est épuisé par deux fois 100 ml d'hexane puis le mélange est évaporé sous vide à sec. Une fois recristallisés dans l'hexane, les esters d'acides aminés sont obtenus sous forme de chlorhydrate. La masse obtenue est de 136,8 g ce qui correspond à un rendement d'estérification par rapport aux acides aminés de 94 %.

### (3) Hydrolyse enzymatique sélective de la position α des diesters des acides L-glutamique et L-aspartique

Les 136,8 g ou 0,69 mole d'esters d'acides aminés de fibroïne de soie renfermant les di-esters des acides L-glutamique et L-aspartique (0,58 mole) sont repris dans un erlenmeyer de 500 ml et additionnés de 20 ml de méthanol puis de 200 ml de tampon phosphate à 0,01 M afin de maintenir le pH du mélange à la neutralité (pH = 7). Le mélange est agité à température ambiante puis additionné sous agitation de 46,0 g de PLE (*Porcine Liver Esterase* 15 U/mg, Sigma E 3019) soit environ 690.000 unités. Le mélange est agité durant 5 heures. Le pH est maintenu à 7 par ajout d'une solution d'hydroxyde de sodium à 1M. Lorsque un équivalent de base est consommé, la réaction est stoppée avec de l'acide chlorhydrique (environ 1M) par acidification du milieu à pH 3-4. Le mélange est additionné de 50 ml de méthanol. Les esters d'acides aminés renfermant les esters des acides glutamique et aspartique hydrolysés sélectivement en position α précipitent avec les autres mono-esters. Ils sont ensuite isolés par filtration puis séchés à 40°C à l'étuve ventilée. La masse d'acides aminés mono-estérifiés obtenue est de 127,2 g, ce qui correspond à un rendement de réaction de 93 % soit 0,64 mole.

### (4) Condensation du chlorure de palmitoyle et des acides aminés mono-estérifiés

Les 127,2 g (0,64 mole) d'acides aminés mono-estérifiés pris sous forme de chlorhydrates sont placés dans un réacteur de un litre en présence de 400 ml de chlorure de méthylène et de 1,6 moles de triéthylamine soit 223 ml (2,5 équivalents / chlorhydrates d'esters d'acides aminés). Le mélange est refroidi entre 0-5°C puis additionné de 0,95 équivalent soit 0,61 mole ou 167,6 g de chlorure de palmitoyle en solution dans 30 ml de chlorure de méthylène. Après addition de l'agent acylant, le mélange est agité à 35°C pendant quatre heures puis transféré dans une ampoule à décanter. La phase organique est lavée successivement quatre fois par 450 ml d'eau acidulée pour éliminer la triéthylamine en excès. La phase organique est séchée sur sulfate de sodium, filtrée puis évaporée sous vide. La masse de N-palmitoyl-L-glutamate-γ-methyl-ester d'hydrolysat de fibroïne de soie enrichie obtenue est de 256 g, ce qui correspond à un rendement massique de condensation de 90 %.

### (5) Salification du N-palmitoyl-L-glutamate-γ-methyl-ester

256 g de N-palmitoyl-L-glutamate-γ-methyl-ester d'hydrolysat de fibroïne de soie enrichi soit 0,55 mole sont placés dans un réacteur de un litre et solubilisés dans 300 ml de méthanol. Le mélange est additionné d'une quantité équimolaire d'une solution aqueuse d'hydroxyde de sodium (par rapport aux fonctions acides α-carboxyliques libres, soit 0,43 mole), soit 0,43 mole de NaOH soit 17,2 g. Le mélange est agité durant deux heures à température ambiante, ce qui conduit essentiellement à l'obtention de sels mono sodiques du N-palmitoyl-L-glutamate-γ-methyl-ester et dans de très faibles proportions de sels mono sodiques du N-palmitoyl-L-aspartate-β-methyl-ester. Les N-palmitoylamino mono-esters issus de l'hydrolysat de fibroïne de soie ne sont pas concernés par l'étape de salification.

### (6) Substitution des sels mono sodiques du N-palmitoyl-L-glutamate-γ-methyl-ester de fibroïne de soie avec le zinc

Les sels mono sodiques du N-palmitoyl-L-glutamate-γ-methyl-ester d'hydrolysat de fibroïne de soie sont substitués par le zinc par ajout d'une solution aqueuse de chlorure de zinc réalisée à partir de 0,5 équivalent de ZnCl₂ soit 0,22 mole et 29,3 g. Une fois la solution de chlorure de zinc additionnée, les sels de zinc di-palmitoyl-L-glutamate-γ-methyl-ester de fibroïne de soie précipitent totalement dans le milieu en association avec les autres N-acylamino esters présents dans le mélange. Le pH est amené à 3-4, puis les sels de zinc sont filtrés sur fritté sous vide, lavés à l'eau puis séchés à l'étuve ventilée à 40°C jusqu'à l'obtention d'une poudre blanche. Les sels de zinc di-(N-palmitoyl-L-glutamate-γ-methyl-ester) associés aux N-palmitoylamino mono-esters issus de l'hydrolysat de fibroïne de soie sont obtenus avec une masse de 221,7 g soit rendement massique de substitution de 92 %. D'une manière générale, le rendement global de la synthèse est de 73 % (m/m) et les produits obtenus présentent un degré de pureté supérieur à 90 %.

### Exemple 4

Synthèse de sels de calcium di-(N-palmitoyl-L-glutamate-γ-methyl-ester) d'hydrolysat de fibroïne de soie.

Les étapes de synthèse un à cinq pour la réalisation des sels de calcium de di-(N-pahnitoyl-L-glutamate-γ-methyl-ester) d'hydrolysat de fibroïne de soie sont identiques à celles décrites dans l'exemple 3 à l'exception de la dernière étape (étape 6) qui est détaillée ci-après :

### (6) Substitution des sels mono sodiques du N-palmitoyl-L-glutamate-γ-methyl-ester de fibroïne de soie avec le calcium

Les sels mono sodiques du N-palmitoyl-L-glutamate-γ-methyl-ester de fibroïne de soie sont complexés avec le calcium par ajout d'une solution aqueuse de chlorure de calcium réalisée à partir de 0,5 équivalent de CaCl₂ soit 0,24 mole et 26,6 g. Une fois la solution de chlorure de calcium additionnée, les sels de calcium di-(N-palmitoyl-L-glutamate-γ-methyl-ester) de fibroïne de soie précipitent totalement dans le milieu en association avec les autres N-acylamino esters présents dans le mélange. Le pH est amené à 3-4, puis les sels de calcium sont filtrés sur fritté sous vide, lavés à l'eau puis séchés à l'étuve ventilée à 40°C jusqu'à l'obtention d'une poudre blanche. Les sels de calcium di-(N-palmitoyl-L-glutamate-γ-methyl-ester) associés aux N-palmitoylamino mono-esters issus de l'hydrolysat de fibroïne de soie sont obtenus avec une masse de 216,1 g soit rendement massique de substitution de 90 %.

D'une manière générale, le rendement global de la synthèse est de 73 % (m/m) et les produits obtenus présentent un degré de pureté supérieur à 90 %.

### Exemple 5

Synthèse de sels d'aluminium tri-(N-palmitoyl-L-glutamate-γ-methyl-ester) de caséine de lait de vache

### (1) Préparation des acides aminés de caséine enrichi en acide L-glutamique

Dans un erlenmeyer, 20 g d'acides aminés de caséine atomisée sont additionnés de 80 g d'acide L-glutamique selon les proportions massiques correspondants au mélange binaire 20 % d'acides aminés de caséine et 80 % d'acide L-glutamique. La composition en acides aminés de la caséine qui est utilisée est donnée dans le tableau 3.

**Tableau 3**

| Aminogramme des acides aminés atomisés de la caséine (% m/m) | | | |
|---|---|---|---|
| Acides aminés | % massiques | Acides aminés | % massiques |
| Alanine | 2.9 | Lysine | 7.0 |
| Arginine | 3.6 | Méthionine | 2.7 |
| A. Aspartique | 6.5 | Phénylalanine | 4.7 |
| Cystine | 0.2 | Proline | 10.4 |
| A. Glutamique | 20.5 | Sérine | 5.9 |
| Glycine | 1.7 | Thréonine | 3.8 |
| Histidine | 2.7 | Tryptophane | 1.2 |
| Isoleucine | 5.8 | Tyrosine | 5.1 |
| Leucine | 8.8 | Valine | 6.5 |

La proportion initiale d'acides aminés di-acides présents naturellement dans la caséine est élevée avec un pourcentage global de 27 %, majoritairement représenté par l'acide L-glutamique (20,5 %). L'addition de 80 g d'acide L-glutamique à 20 g d'hydrolysat de caséine permet d'augmenter artificiellement les proportions globales d'acide L-glutamique et de disposer d'un hydrolysat de caséine fortement enrichi en acides aminés di-acides. La composition en acides aminés de la caséine enrichie en acide L-glutamique est donnée dans le tableau 4.

**Tableau 4**

| Pourcentages massiques des acides aminés de caséine atomisés et enrichis en acide L-glutamique (20/80, m/m) | | | |
|---|---|---|---|
| Acides aminés | % massiques | Acides aminés | % massiques |
| Alanine | 0.6 | Lysine | 1.4 |
| Arginine | 0.7 | Méthionine | 0.5 |
| A. Aspartique | 1.3 | Phénylalanine | 1.0 |
| Cystine | Traces | Proline | 2.1 |
| A. Glutamique (total) | 84.1 | Sérine | 1.2 |
| Glycine | 0.3 | Thréonine | 0.8 |
| Histidine | 0.6 | Tryptophane | 0.1 |
| Isoleucine | 1.2 | Tyrosine | 1.0 |
| Leucine | 1.8 | Valine | 1.3 |

Une fois enrichi, le pourcentage massique total en acide L-glutamique est de 84,1 % auquel il faut rajouter la masse de 1,3 g d'acide L-aspartique, soit une masse globale de di-acides dans le mélange de 85,4 g. Le mélange protéique obtenu est ensuite méthyl estérifié.

### (2) Estérification de l'hydrolysat de protéine de caséine enrichi en acide L-glutamique

L'estérification de 100 g d'hydrolysat de caséine enrichi en acide L-glutamique est réalisée dans un ballon bicol de 500 ml. Les équivalences de réactifs à additionner sont calculées suivant le nombre total de moles d'acides aminés à estérifier. Le tableau 5 présente les pourcentages molaires de l'hydrolysat de caséine atomisé enrichi en acide L-glutamique.

**Tableau 5**

| Pourcentages molaires des acides aminés de caséine atomisés et enrichis en acide L-glutamique (20/80, m/m) | | | |
|---|---|---|---|
| Acides aminés | % molaires | Acides aminés | % molaires |
| Alanine | 0.007 | Lysine | 0.010 |
| Arginine | 0.004 | Méthionine | 0.003 |
| A. Aspartique | 0.010 | Phénylalanine | 0.006 |
| Cystine | --- | Proline | 0.018 |
| A. Glutamique | 0.572 | Sérine | 0.011 |
| Glycine | 0.004 | Thréonine | 0.007 |
| Histidine | 0.004 | Tryptophane | 0.001 |
| Isoleucine | 0.009 | Tyrosine | 0.006 |
| Leucine | 0.014 | Valine | 0.011 |

Sur les 100 g mis en jeux, le nombre total de moles à estérifier est égal à 0,70 (0,11 mole d'acides aminés mono-acides et 0,58 mole d'acide aminés di-acides, soit environ 83 %). Le mélange d'acides aminés est additionné de 350 ml de méthanol puis de 2,2 équivalents de SOCl₂ pour les di-acides, soit 1,3 moles (151,8 g) et 1,2 équivalents de SOCl₂ pour les mono-acides, soit 0,13 mole (15,7 g) ce qui ramène à une masse totale de SOCl₂ de 167,5 g et un volume de 103 ml. Le chlorure de thionyle est additionné goutte à goutte tout en maintenant le mélange méthanolique d'acides aminés à une température proche de 5°C (bain de glace). Lorsque la totalité du SOCl₂ a été additionnée, le mélange réactionnel est ensuite chauffé à reflux pendant deux heures. Le méthanol et le SOCl₂ en excès sont éliminés à l'évaporateur rotatif sous vide. Le méthanol résiduel est épuisé par deux fois 100 ml d'hexane puis le mélange est évaporé sous vide à sec. Une fois recristallisés dans l'hexane, les esters d'acides aminés sont obtenus sous forme de chlorhydrate. La masse obtenue est de 135 g ce qui correspond à un rendement d'estérification par rapport aux acides aminés de 94 %.

### (3) Hydrolyse enzymatique sélective de la position α des diesters des acides L-glutamique et L-aspartique

Les 135 g ou 0,66 mole d'esters d'acides aminés de caséine renfermant les di-esters des acides L-glutamique et L-aspartique (0,54 mole) sont repris dans un erlenmeyer de 500 ml et additionnés de 20 ml de méthanol puis de 200 ml de tampon phosphate à 0,01 M afin de maintenir le pH du mélange à la neutralité (pH = 7). Le mélange est agité à température ambiante puis additionné sous agitation de 46,0 g de PLE (*Porcine Liver Esterase* 15 U/mg, Sigma E 3019) soit environ 690.000 unités. Le mélange est agité durant 5 heures. Le pH est maintenu à 7 par ajout d'une solution d'hydroxyde de sodium à 1M. Lorsque un équivalent de base est consommé, la réaction est stoppée avec de l'acide chlorhydrique (environ 1M) par acidification du milieu à pH 3-4. Le mélange est additionné de 50 ml de méthanol. Les esters d'acides aminés renfermant les esters des acides glutamique et aspartiques hydrolysés sélectivement en position α précipitent avec les autres mono-esters. Ils sont ensuite isolés par filtration puis séchés à 40°C à l'étuve ventilée. La masse d'acides aminés mono-estérifiés obtenue est de 125,5 g, ce qui correspond à un rendement de réaction de 93 % soit 0,61 mole.

### (4) Condensation du chlorure de palmitoyle et des acides aminés mono-estérifiés

Les 125,5 g (0,61 mole) d'acides aminés mono-estérifiés pris sous forme de chlorhydrates sont placés dans un réacteur de un litre en présence de 400 ml de chlorure de méthylène et de 1,5 moles de triéthylamine soit 212,5 ml (2,5 équivalents / chlorhydrates d'esters d'acides aminés). Le mélange est refroidi entre 0-5°C puis additionné de 0,95 équivalent soit 0,58 mole ou 159,1 g de chlorure de palmitoyle en solution dans 30 ml de chlorure de méthylène. Après addition de l'agent acylant, le mélange est agité à 35°C pendant quatre heures puis transféré dans une ampoule à décanter. La phase organique est lavée successivement quatre fois par 450 ml d'eau acidulée pour éliminer la triéthylamine en excès. La phase organique est séchée sur sulfate de sodium, filtrée puis évaporée sous vide. La masse de N-palmitoyl-L-glutamate-γ-methyl-ester de caséine enrichie obtenue est de 247,8 g, ce qui correspond à un rendement massique de condensation de 90 %.

### (5) Salification du N-palmitoyl-L-glutamate-γ-methyl-ester de caséine enrichie

Les 247,8 g de N-palmitoyl-L-glutamate-γ-methyl-ester de caséine soit 0,55 mole sont placés dans un réacteur de un litre et solubilisés dans 300 ml de méthanol. Le mélange est additionné d'une quantité équimolaire d'une solution aqueuse d'hydroxyde de sodium (par rapport aux fonctions acides α-carboxyliques libres, soit 0,48 mole), soit 0,48 mole de NaOH soit 19 g. Le mélange est agité durant deux heures à température ambiante, ce qui conduit essentiellement à l'obtention de sels mono sodiques du N-palmitoyl-L-glutamate-γ-methyl-ester et dans de très faibles proportions de sels mono sodiques du N-palmitoyl-L-aspartate-β-methyl-ester. Les N-palmitoyl amino mono-esters issus de l'hydrolysat de caséine ne sont pas concernés par l'étape de salification.

### (6) Substitution des sels mono sodiques du N-palmitoyl-L-glutamate-γ-methyl-ester de caséine par l'aluminium

Les 0,48 mole de sels mono sodiques du N-palmitoyl-L-glutamate-γ-methyl-ester et du N-palmitoyl-L-aspartate-β-methyl-ester de caséine_sont substitués par l'aluminium par ajout d'une solution aqueuse de chlorure d'aluminium hexahydraté (AlCl₃ 6H₂O) réalisée à partir de 0,33 équivalent de AlCl₃ 6H₂O soit 0,16 mole et 38,6 g. Une fois la solution de chlorure d'aluminium hexahydraté additionnée, les sels d'aluminium tri-(N-palmitoyl-L-glutamate-γ-methyl-ester) et tri-(N-palmitoyl-L-aspartate-β-methyl-ester) de caséine précipitent totalement dans le milieu en association avec les autres N-acylamino esters présents dans le mélange. Le pH est amené à 3-4, puis les sels d'aluminium sont filtrés sur fritté sous vide, lavés à l'eau puis séchés à l'étuve ventilée à 40°C jusqu'à l'obtention d'une poudre blanche. Les sels d'aluminium tri-(N-palmitoyl-L-glutamate-γ-methyl-ester) et tri-(N-palmitoyl-L-aspartate-β-methyl-ester) associés aux N-palmitoylamino mono-esters issus de l'hydrolysat de caséine sont obtenus avec une masse de 204,4 g soit rendement massique de substitution de 92 %. D'une manière générale, le rendement global de la synthèse est de 73 % (m/m) et les produits obtenus présentent un degré de pureté supérieur à 90 %.

### Exemple 6

### Synthèse de sels de magnésium di-(N-palmitoyl-L-glutamate-γ-methyl-ester) d'hydrolysat de caséine de lait de vache

Les étapes de synthèse un à cinq pour la réalisation des sels de magnésium de di-(N-palmitoyl-L-glutamate-γ-methyl-ester) d'hydrolysat de caséine de lait de vache sont identiques à celles décrites dans l'exemple 5 à l'exception de la dernière étape (étape 6) qui est détaillée ci-après :

### (6) Substitution des sels mono sodiques du N-palmitoyl-L-glutamate-γ-methyl-ester de caséine par le magnésium

Les 0,48 mole de sels mono sodiques du N-palmitoyl-L-glutamate-γ-methyl-ester et du N-palmitoyl-L-aspartate-β-methyl-ester de caséine sont substitués par le magnésium par ajout d'une solution aqueuse de chlorure de magnésium (MgCl₂) réalisée à partir de 0,5 équivalent de MgCl₂ soit 0,24 mole et 22,8 g. Une fois la solution de chlorure de magnésium additionnée, les sels de magnésium di-(N-palmitoyl-L-glutamate-γ-methyl-ester) et di-(N-palmitoyl-L-aspartate-β-methyl-ester) d'hydrolysat de caséine précipitent totalement dans le milieu en association avec les autres N-acylamino esters présents dans le mélange. Le pH est amené à 3-4, puis les sels de magnésium sont filtrés sur fritté sous vide, lavés à l'eau puis séchés à l'étuve ventilée à 40°C jusqu'à l'obtention d'une poudre blanche. Les sels de magnésium di-(N-palmitoyl-L-glutamate-γ-methyl-ester) et di-(N-palmitoyl-L-aspartate-β-methyl-ester) associés aux N-palmitoylamino mono-esters issus de l'hydrolysat de caséine sont obtenus avec une masse de 205,7 g soit rendement massique de complexation de 92 %.

D'une manière générale, le rendement global de la synthèse est de 73 % (m/m) et les produits obtenus présentent un degré de pureté supérieur à 90 %.

### Exemple 7

### Synthèse des sels de sélénium de tétra-(N-palmitoyl- L-aspartate-β-methyl-ester/L-glutamate-γ-methyl-ester) d'avoine

### (1) Préparation de l'hydrolysat de protéine d'avoine

Dans un erlenmeyer, peser 100 g d'acides aminés d'avoine atomisée. La composition en acides aminés de l'hydrolysat d'avoine utilisé est donné dans le tableau 6.

**Tableau 6**

| Aminogramme des acides aminés atomisés d'avoine (% m/m) | | | |
|---|---|---|---|
| Acides aminés | % massiques | Acides aminés | % massiques |
| Alanine | 4.8 | Lysine | 3.9 |
| Arginine | 6.7 | Méthionine | 1.9 |
| A. Aspartique | 8.4 | Phénylalanine | 5.3 |
| Cystéine | 2.9 | Proline | 5.5 |
| A. Glutamique | 22.6 | Sérine | 5.0 |
| Glycine | 5.0 | Thréonine | 3.6 |
| Histidine | 2.2 | Tryptophane | 1.3 |
| Isoleucine | 4.1 | Tyrosine | 3.6 |
| Leucine | 7.7 | Valine | 5.5 |

La proportion initiale d'acides aminés di-acides présents naturellement dans l'hydrolysat d'avoine est élevée avec un pourcentage massique global de 31 %, majoritairement représenté par l'acide L-glutamique (22,6 %). La composition molaire en acides aminés de l'hydrolysat d'avoine est donnée dans le tableau 7.

**Tableau 7**

| Pourcentages molaires des acides aminés de l'hydrolysat d'avoine | | | |
|---|---|---|---|
| Acides aminés | % molaires | Acides aminés | % molaires |
| Alanine | 0.05 | Lysine | 0.03 |
| Arginine | 0.04 | Méthionine | 0.01 |
| A. Aspartique | 0.06 | Phénylalanine | 0.03 |
| Cystine | 0.01 | Proline | 0.05 |
| A. Glutamique | 0.15 | Sérine | 0.05 |
| Glycine | 0.07 | Thréonine | 0.03 |
| Histidine | 0.01 | Tryptophane | 0.01 |
| Isoleucine | 0.03 | Tyrosine | 0.02 |
| Leucine | 0.06 | Valine | 0.05 |

Le nombre total de mole d'acides aminés provenant de 100 g d'hydrolysat d'avoine est de 0,76 mole. mélange protéique obtenu est ensuite méthyl estérifié.

### (2) Estérification de l'hydrolysat de protéines d'avoine enrichi en acide L-glutamique

Sur les 100 g d'hydrolysat d'avoine mis en jeux, le nombre total de moles à estérifier est égal à 0,76 (0,55 mole d'acides aminés mono-acides et 0,21 mole d'acide aminés di-acides, soit environ 28 %). Le mélange d'acides aminés est additionné de 350 ml de méthanol puis de 2,2 équivalents de SOCl₂ pour les di-acides, soit 0,46 mole (54,7 g) et 1,2 équivalents de SOCl₂ pour les mono-acides, soit 0,66 mole (78,5 g) ce qui ramène à une masse totale de SOCl₂ de 133,2 g et un volume de 82 ml. Le chlorure de thionyle est additionné goutte à goutte tout en maintenant le mélange méthanolique d'acides aminés à une température proche de 5°C (bain de glace). Lorsque la totalité du SOCl₂ a été additionnée, le mélange réactionnel est ensuite chauffé à reflux pendant deux heures. Le méthanol et le SOCl₂ en excès sont éliminés à l'évaporateur rotatif sous vide. Le méthanol résiduel est épuisé par deux fois 100 ml d'hexane puis le mélange est évaporé sous vide à sec. Une fois recristallisés dans l'hexane, les esters d'acides aminés sont obtenus sous forme de chlorhydrate. La masse obtenue est de 135,6 g ce qui correspond à un rendement d'estérification par rapport aux acides aminés d'environ 94 %.

### (3) Hydrolyse enzymatique sélective de la position α des diesters des acides L-glutamique et L-aspartique

Les 135,6 g ou 0,71 mole d'esters d'acides aminés d'avoine renfermant les di-esters des acides L-glutamique et L-aspartique (0,20 mole) sont repris dans un erlenmeyer de 500 ml et additionnés de 10 ml de méthanol puis de 200 ml de tampon phosphate à 0,01 M afin de maintenir le pH du mélange à la neutralité (pH = 7). Le mélange est agité à température ambiante puis additionné sous agitation de 13,0 g de PLE (*Porcine Liver Esterase* 15 U/mg, Sigma E 3019) soit environ 200.000 unités. Le mélange est agité durant 5 heures. Le pH est maintenu à 7 par ajout d'une solution d'hydroxyde de sodium à 1M. Lorsque un équivalent de base est consommé, la réaction est stoppée avec de l'acide chlorhydrique (environ 1M) par acidification du milieu à pH 3-4. Le mélange est additionné de 50 ml de méthanol. Les esters d'acides aminés renfermant les esters des acides glutamique et aspartiques hydrolysés sélectivement en position α précipitent avec les autres mono-esters. Ils sont ensuite isolés par filtration puis séchés à 40°C à l'étuve ventilée. La masse d'acides aminés mono-estérifiés obtenue est de 126,1 g, ce qui correspond à un rendement de réaction de 93 % soit 0,66 mole.

### (4) Condensation du chlorure de palmitoyle et des acides aminés mono-estérifiés

Les 126,1 g (0,66 mole) d'acides aminés mono-estérifiés pris sous forme de chlorhydrates sont placés dans un réacteur de un litre en présence de 400 ml de chlorure de méthylène et de 1,65 moles de triéthylamine soit 230 ml (2,5 équivalents / chlorhydrates d'esters d'acides aminés). Le mélange est refroidi entre 0-5°C puis additionné de 0,95 équivalent soit 0,62 mole ou 172,2 g de chlorure de palmitoyle en solution dans 30 ml de chlorure de méthylène. Après addition de l'agent acylant, le mélange est agité à 35°C pendant quatre heures puis transféré dans une ampoule à décanter. La phase organique est lavée successivement quatre fois par 450 ml d'eau acidulée pour éliminer la triéthylamine en excès. La phase organique est séchée sur sulfate de sodium, filtrée puis évaporée sous vide. La masse de N-palmitoyl-amino esters d'avoine renfermant 31 % de fonctions acides en position α est de 218,3 g, ce qui correspond à un rendement massique de condensation d'environ 90 %.

### (5) Salification des fonctions acides en position α des N-palmitoyl-amino esters d'avoine

Les 218,3 g de N-palmitoyl-amino esters d'avoine soit 0,60 mole sont placés dans un réacteur de un litre et solubilisés dans 300 ml de méthanol. Le mélange est additionné d'une quantité équimolaire d'une solution aqueuse d'hydroxyde de sodium (par rapport aux fonctions acides α-carboxyliques libres, soit 0,17 mole), soit 0,17 mole de NaOH soit 6,8 g. Le mélange est agité durant deux heures à température ambiante, ce qui conduit essentiellement à l'obtention de sels mono sodiques des N-palmitoyl-L-glutamate-γ-methyl-ester et des N-palmitoyl-L-aspartate-β-methyl-ester. Les autres N-palmitoyl amino mono-esters (soit 0,43 mole) ne sont pas concernés par l'étape de salification.

### (6) Substitution des sels mono sodiques du N-palmitoyl-L-glutamate-γ-methyl-ester et du N-palmitoyl-L-aspartate-β-methyl-ester d'avoine par le sélénium

Les 0,17 mole de sels mono sodiques du N-palmitoyl-L-glutamate-γ-methyl-ester et du N-palmitoyl-L-aspartate-β-methyl-ester d'avoine sont substitués par le sélénium par ajout d'une solution aqueuse de tétrachlorure de sélénium (SeCl₄) réalisée à partir de 0,25 équivalent de SeCl₄ soit 0,042 mole et 9,3 g. Une fois la solution de tétrachlorure de sélénium additionnée, les sels de sélénium tétra-(N-palmitoyl-L-glutamate-γ-methyl-ester) et tétra-(N-palmitoyl-L-aspartate-β-methyl-ester) d'avoine précipitent totalement dans le milieu en association avec les autres N-acylamino esters présents dans le mélange. Le pH est amené à 3-4, puis les sels de sélénium sont filtrés sur fritté sous vide, lavés à l'eau puis séchés à l'étuve ventilée à 40°C jusqu'à l'obtention d'une poudre blanche. Les sels de sélénium tétra-(N-pahnitoyl-L-glutamate-γ-methyl-ester) et tétra-(N-pahnitoyl-L-aspartate-β-methyl-ester) associés aux N-palmitoylamino mono-esters issus de l'hydrolysat d'avoine sont obtenus avec une masse de 216,2 g soit rendement massique de substitution de 92 %. D'une manière générale, le rendement global de la synthèse est de 73 % (m/m) et les produits obtenus présentent un degré de pureté supérieur à 90 %.

Les sels de N-acyl-L-aspartate-β-mono-ester et les sels de N-acyl-L-glutamate-γ-mono-ester obtenus sont destinés à être incorporés dans des compositions pharmaceutiques, cosmétiques et dermatologiques selon des proportions massiques qui varient de 0,1 à 30 % selon les besoins. Des compositions de préparations galéniques sont donnés ci-après à titre d'exemple.

### • Composition crème apaisante peaux sensibles

| **INGREDIENTS** | **% (w/w)** |
|---|---|
| ISOPROPYL PALMITATE | 15,0 |
| GLYCERYL STEARATE | 5,0 |
| CETEARETH-20 | 3,0 |
| LANOLIN | 2,0 |
| GLYCERIN | 10,0 |
| **Calcium di-(N-palmitoyl-L-glutamate-γ-methyl-ester) de fibroïne de soie** | 2,0 |
| **Zinc di-(N-palmitoyl-L-aspartate-β-methyl-ester)** | 3,0 |
| ALPHA TOCOPHÉROL | 0,5 |
| PARFUM (FRAGRANCE) | 0,2 |
| CONSERVATEURS (Antiseptics) | Quantité nécessaire |
| AQUA (Purified water) | qsp 100 (balance) |

### • Composition gel purifiant peaux grasses et acnéiques

| **INGREDIENTS** | **% (w/w)** |
|---|---|
| CAPRYLIC / CAPRIC TRIGLYCERIDE | 3,0 |
| CYCLOHEXASILOXANE | 6,0 |
| GLYCERIN | 5,0 |
| **Cuivre di-(N-palmitoyl-L-glutamate-γ-methyl-ester)** | 0,4 |
| **Zinc di-(N-palmitoyl-L-aspartate-β-methyl-ester)** | 2,0 |
| POLYACRYLAMIDE (and) C13-14 ISOPARAFFIN (and) LAURETH-7 | 3,0 |
| PARFUM (FRAGRANCE) | Quantité nécessaire |
| CONSERVATEURS (Antiseptics) | Quantité nécessaire |
| AQUA (Purified water) | qsp 100 (balance) |

### • Composition crème antitranspirante pieds peaux sensibles

| **INGREDIENTS** | **% (w/w)** |
|---|---|
| GLYCÉRYL STÉARATE | 7,0 |
| GLYCÉRINE | 5,0 |
| CETEARETH-12 | 4,0 |
| PARAFFINUM LIQUIDUM | 3,0 |
| ALUMINIUM CHLORHYDRATE | 10,0 |
| **Aluminium tri-(N-palmitoyl-L-glutamate-γ-methyl-ester) de caséine de lait de vache** | 5,0 |
| ALPHA TOCOPHÉROL | 0,2 |
| FRAGRANCE | Quantité nécessaire |
| CONSERVATEURS (Antiseptics) | Quantité nécessaire |
| AQUA (Purified water) | qsp 100 (balance) |

### Composition savon crème purifiant peaux grasses et acnéiques

| **INGRÉDIENTS** | **% (w/w)** |
|---|---|
| GLYCÉRYL STÉARATE | 8,0 |
| PARAFFINUM LIQUIDUM | 4,0 |
| SODIUM TEA HYDROLIZED PROTEIN | 3,0 |
| **Zinc di-(N-palmitoyl-L-aspartate-β-methyl-ester)** | 1,5 |
| **Cuivre di-(N-palmitoyl-L-glutamate-γ-methyl-ester)** | 0,5 |
| STEARIC ACID | Quantité nécessaire |
| CONSERVATEURS (Antiseptics) | Quantité nécessaire |
| FRAGRANCE | Quantité nécessaire |
| AQUA (Purified water) | qsp 100 (balance) |

### • Composition shampooing apaisant cuir chevelu sensibilisé

| **INGRÉDIENTS** | **% (w/w)** |
|---|---|
| SODIUM COCETH SULFATE | 10,0 |
| SODIUM LAURYL SULFATE | 15,0 |
| COCOAMIDOPROPYL BETAÏNE | 7,0 |
| PEG 40 GLYCERYL COCOATE | 4,0 |
| COCAMIDE DEA | 3,0 |
| **Zinc di-(N-palmitoyl-L-aspartate-β-methyl-ester)** | 2,0 |
| **Cuivre di-(N-palmitoyl-L-glutamate-γ-methyl-ester)** | 1,0 |
| FRAGRANCE | Quantité nécessaire |
| CONSERVATEURS (Antiseptics) | Quantité nécessaire |
| AQUA (PURIFIED WATER) | qsp 100 (balance) |

### • Composition lotion cheveux gras

| **INGRÉDIENTS** | **% (w/w)** |
|---|---|
| ALCOOL DÉNATURÉ | 45,0 |
| **Zinc di-(N-palmitoyl-L-aspartate-β-methyl-ester) de fibroïne de soie** | 0,5 |
| **Cuivre di-(N-palmitoyl-L-aspartate-β-methyl-ester) de fibroïne de soie** | 1,0 |
| ROSMARINUS OFFICINALIS | 0,2 |
| THYMUS VULGARIS | 1,0 |
| AQUA (PURIFIED WATER) | qsp 100 (balance) |

### • Composition sérum antirides

| **INGRÉDIENTS** | **% (w/w)** |
|---|---|
| CAMELINA SATIVA | 5,0 |
| CAPRYLIC / CAPRIC TRIGLYCERIDE | 10,0 |
| GLYCERINE | 6,0 |
| RETiNYL PALMITATE | 0,5 |
| TOCOPHERYL ACETATE | 1,0 |
| POLYACRYLAMIDE (and) C13-14 ISOPARAFFIN (and) LAURETH-7 | 3,0 |
| **Calcium di-(N-palmitoyl-L-aspartate-β-methyl-ester)** | 2,0 |
| **Selenium di-(N-palmitoyl-L-glutamate-γ-methyl-ester)** | 0,5 |
| FRAGRANCE | Quantité nécessaire |
| CONSERVATEURS (Antiseptics) | Quantité nécessaire |
| AQUA (PURIFIED WATER) | qsp 100 (balance) |

## Revendications

1. Sels de N-acyl-L-aspartate-β-mono-ester et/ou de N-acyl-L-glutamate-γ-mono-ester ayant la formule générale suivante : 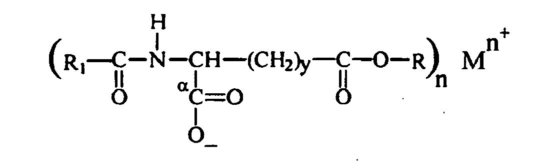 dans laquelle Mⁿ⁺ représente un métal ou un oligo-élément **caractérisé par** un état de valence n supérieur à l'unité et inférieur ou égal à 7, R₁ représente un radical alkyle, aryle, saturé ou insaturé de condensation ayant de 1 à 30 atomes de carbone, R est choisi parmi la partie hydrocarbone d'alcools linéaires ou ramifiés, cycliques ou acycliques et y est égal à 1 pour l'acide aspartique et est égal à 2 pour l'acide glutamique.

2. Procédé de préparation des sels selon la revendication 1, comprenant les étapes suivantes :
- l'estérification par un alcool linéaire ou ramifié du ou des acides aminés di-acides L-aspartique et L-glutamique et éventuellement des acides aminés mono-acides associés provenant des hydrolysats de protéines ;
- l'hydrolyse sélective par voie chimique ou enzymatique des fonctions esters situées en α des di-esters des acides aminés L-aspartique et L-glutamique présents dans le mélange ;
- la N-acylation en milieu organique de tous les esters d'acides aminés présents dans le mélange par un halogénure d'acide alkyle, aryle saturé ou non ;
- la salification par des bases mono-sodiques ou mono-potassiques de la fonction acide carboxylique libre située en α des dérivés N-acyl-L-aspartique-β-mono-ester et des dérivés N-acyl-L-glutamique-γ-mono-ester de façon à obtenir les sels mono-sodiques ou mono-potassiques correspondants ;
ce procédé étant **caractérisé par** l'étape suivante :
- la substitution des sels alcalins mono-sodiques ou mono-potassiques des N-acyl-L-aspartate-β-mono-ester et N-acyl-L-glutamate-γ-mono-ester par des sels métalliques bivalents, trivalents, tétravalents, pentavalents, hexavalents ou heptavalents provenant de métaux, d'alcalino-terreux, de métaux de transition incluant les lanthanides et les actinides.

3. Sels selon la revendication 1 ou obtenus par le procédé selon la revendication 2, **caractérisés en ce que** les métaux complexants Mⁿ⁺ sont choisis parmi les cations bivalents (n=2) suivants : le zinc, le cuivre, le nickel, le manganèse, le magnésium, le calcium, le cobalt, le vanadium, le titane, le chrome, le fer, le silicium, et le cadmium.

4. Sels selon la revendication 1 ou obtenus par le procédé selon la revendication 2, **caractérisés en ce que** les métaux complexants Mⁿ⁺ sont choisis parmi les cations trivalents (n=3) suivants : l'aluminium, le rhodium, le gallium et l'or.

5. Sels selon la revendication 1 ou obtenus par le procédé selon la revendication 2, **caractérisés en ce que** les métaux complexants Mⁿ⁺ sont choisis parmi les cations tétravalents (n=4) suivants : le sélénium, le platine et le palladium.

6. Sels selon la revendication 1 ou obtenus par le procédé selon la revendication 2, **caractérisés en ce que** les métaux complexants Mⁿ⁺ sont choisis parmi les cations pentavalents (n=5) suivants : le tantale et le bismuth.

7. Sels selon la revendication 1 ou obtenus par le procédé selon la revendication 2, **caractérisés en ce que** les métaux complexants Mⁿ⁺ sont choisis parmi les cations hexavalents (n=6) comme le molybdène ou parmi les cations heptavalents (n=7) comme le rhénium.

8. Procédé selon la revendication 2, **caractérisé en ce que** les acides aminés di-acides L-aspartique et L-glutamique sont pris individuellement ou en mélange ou bien en combinaison avec des hydrolysats de protéines animales ou végétales ;

9. Procédé selon la revendication 2, **caractérisé en ce que** les hydrolysats de protéines sont choisis à partir des protéines suivantes : la fibroïne de soie du *Bombyx mori,* le blé, le maïs, le soja, le tournesol, le lupin, la caséine et les algues marines *Laminaria digitata, Ascophyllum nodosum, Fucus vesiculosus, Chondrus crispus, Lithothamnium calcareum, Alsidium helminthocorton* et *Digenea simplex.*

10. Procédé selon la revendication 2, **caractérisé en ce que** les hydrolysats de protéines animales ou végétales peuvent être préalablement enrichis en acides aminés di-acides L-aspartique et/ou L-glutamique dans des proportions massiques pouvant aller respectivement de 5 à 95 % par rapport aux hydrolysats de protéines ;

11. Procédé selon la revendication 2, **caractérisé en ce que** les sels réalisés sont des sels bivalents de zinc di-(N-palmitoyl-L-glutamate-γ-methyl-ester) et des sels bivalents de zinc di-(N-palmitoyl-L-aspartate-β-methyl-ester).

12. Procédé selon la revendication 2, **caractérisé en ce que** les sels réalisés sont des sels bivalents de cuivre di-(N-palmitoyl-L-glutamate-γ-methyl-ester) et des sels bivalents de cuivre di-(N-palmitoyl-L-aspartate-β-methyl-ester).

13. Procédé selon la revendication 2, **caractérisé en ce que** les sels réalisés sont des sels bivalents de zinc di-(N-palmitoyl-L-glutamate-γ-methyl-ester) d'hydrolysats de fibroïne de soie.

14. Procédé selon la revendication 2, **caractérisé en ce que** les sels sont des sels trivalents d'aluminium tri-(N-palmitoyl-L-glutamate-γ-methyl-ester) d'hydrolysats de caséine du lait de vache.

15. Utilisation des sels selon l'une des revendications 1, 3 à 7 ou obtenus par le procédé selon l'une des revendications 2, 8 à 14 dans des compositions pharmaceutiques, cosmétiques ou dermatologiques.

16. Utilisation des sels selon la revendication 15, pour l'obtention de molécules ayant une activité anti-inflammatoire.

17. Utilisation des sels selon la revendication 15, pour l'obtention de molécules ayant une activité anti-microbienne.

18. Utilisation des sels selon la revendication 15, pour l'obtention d'agents anti-sudoraux.

19. Utilisation des sels selon la revendication 15, pour l'obtention de molécules ayant une activité sur la régénération cutanée cellulaire.

20. Utilisation des sels selon la revendication 15, pour l'obtention d'agents anti-radicalaires.

## Claims

1. Salts of N-acyl-L-aspartate β-monoester and/or N-acyl-L-glutamate γ-monoester having the following general formula: 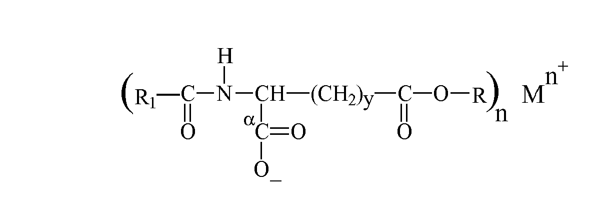 wherein Mⁿ⁺ represents a metal or a trace element **characterized by** a valence state n greater than 1 and less than or equal to 7, R₁ represents a saturated or unsaturated condensed alkyl or aryl radical having from 1 to 30 carbon atoms, R being chosen from among the hydrocarbon portion of linear or branched, or cyclic or acyclic alcohols, and y is equal to 1 for aspartic acid and is equal to 2 for glutamic acid.

2. A method for the preparation of salts according to Claim 1, comprising the following steps:
- esterification of the diacidic amino acids L-aspartic acid and L-glutamic acid and possibly of the associated monoacidic amino acids from protein hydrolysates with a linear or branched alcohol;
- selective hydrolysis by chemical or enzymatic means of the ester functions located at the α-position of the diesters of the amino acids L-aspartic acid and L-glutamic acid present in the mixture;
- N-acylation by a saturated or unsaturated alkyl or aryl acid halide in an organic medium of all of the amino acid esters present in the mixture;
- salt formation by mono-sodium or mono-potassium bases of the free carboxylic acid function located at the α-position of N-acyl-L-aspartate β-monoester derivatives and N-acyl-L-glutamic γ-monoester derivatives in such a manner as to obtain the corresponding mono-sodium or mono-potassium salts;
this method being **characterized by** the following step:
- substitution of the mono-sodium or mono-potassium salts of the N-acyl-L-aspartate β-monoesters and N-acyl-L-glutamic γ-monoesters by bivalent, trivalent, tetravalent, pentavalent, hexavalent or heptavalent metallic salts formed from metals, alkaline earth metals and transition metals including lanthanides and actinides.

3. The salts according to Claim 1 or obtained by the method according to Claim 2, **characterized in that** the complexing metals Mⁿ⁺ are chosen from among the following bivalent cations (n=2): zinc, copper, nickel, manganese, magnesium, calcium, cobalt, vanadium, titanium, chromium, iron, silicon and cadmium.

4. The salts according to Claim 1 or obtained by the method according to Claim 2, **characterized in that** the complexing metals Mⁿ⁺ are chosen from among the following trivalent cations (n=3): aluminum, rhodium, gallium and gold.

5. The salts according to Claim 1 or obtained by the method according to Claim 2, **characterized in that** the complexing metals Mⁿ⁺ are chosen from among the following tetravalent cations (n=4): selenium, platinum and palladium.

6. The salts according to Claim 1 or obtained by the method according to Claim 2, **characterized in that** the complexing metals Mⁿ⁺ are chosen from among the following pentavalent cations (n=5): tantalum and bismuth.

7. The salts according to Claim 1 or obtained by the method according to Claim 2, **characterized in that** the complexing metals Mⁿ⁺ are chosen from among hexavalent cations (n=6) such as molybdenum or from among heptavalent cations (n=7) such as rhenium.

8. The method according to Claim 2, **characterized in that** the diacidic amino acids L-aspartic acid and L-glutamic acid are taken individually or in mixture as well as in combination with animal or vegetable protein hydrolysates.

9. The method according to Claim 2, **characterized in that** the protein hydrolysates are chosen to come from the following proteins: the silk fibroin of *Bombyx mori,* wheat, corn, soya, sunflower, lupine, casein and the marine algae *Laminaria digitata, Ascophyllum nodosum, Fucus vesiculosus, Chondrus crispus, Lithothamnium calcareum, Alsidium helminthocorton* and *Digenea simplex.*

10. The method according to Claim 2, **characterized in that** the animal or vegetable protein hydrolysates can be previously enriched in the diacidic amino acids L-aspartic acid and/or L-glutamic acid in mass ratios from 5% to 95% with respect to the protein hydrolysates.

11. The method according to Claim 2, **characterized in that** the salts produced are the bivalent salt zinc di-(N-palmitoyl-L-glutamate γ-methyl ester) and bivalent salt zinc di-(N-palmitoyl-L-aspartate β-methyl ester).

12. The method according to Claim 2, **characterized in that** the salts produced are the bivalent salt copper di-(N-palmitoyl-L-glutamate γ-methyl ester) and the bivalent salt copper di-(N-palmitoyl-L-aspartate β-methyl ester).

13. The method according to Claim 2, **characterized in that** the salts produced are the bivalent salts of zinc di-(N-palmitoyl-L-glutamate γ-methyl ester) from the silk fibroin hydrolysates.

14. The method according to Claim 2, **characterized in that** the salts produced are the trivalent salts aluminum tri-(N-palmitoyl-L-glutamate γ-methyl ester) from casein hydrolysates from cow's milk.

15. Use of the salts according to one of the Claims 1, 3 to 7 or obtained by the method according to one of the Claims 2 ,8 to 14 in pharmaceutical, cosmetic or dermatological compositions.

16. Use of the salts according to Claim 15 to obtain molecules that have antiinflammatory activity.

17. Use of the salts according to Claim 15 to obtain molecules that have antimicrobial activity.

18. Use of the salts according to Claim 15 to obtain anti-perspiration agents.

19. Use of the salts according to Claim 15 to obtain molecules that have activity on cutaneous cellular regeneration.

20. Use of the salts according to Claim 15 to obtain antiradical agents.

## Patentansprüche

1. Salze von β-Monoestern der N-Acyl-L-asparaginsäure und/oder von γ-Monoestem der N-Acyl-L-glutaminsäure nach der folgenden allgemeinen Formel: 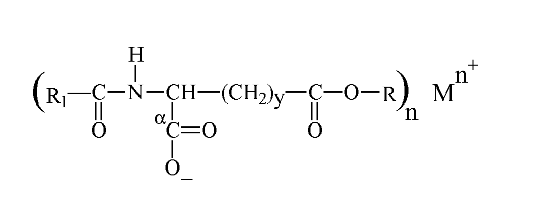 wobei Mⁿ⁺ für ein Metall oder ein Oligoelement steht, welches durch eine Wertigkeit n **gekennzeichnet** ist, die größer als 1 ist und höchstens 7 beträgt, wobei R₁ für einen gesättigten oder ungesättigten Alkyl- oder Arylrest steht, der aus einer Kondensationsreaktion stammt und 1 bis 30 Kohlenstoffatome umfasst, und R für den Kohlenwasserstoffteil von geradkettigen oder verzweigten, ringförmigen oder offenkettigen, Alkoholen steht und y für Asparaginsäure gleich 1 und für Glutaminsäure gleich 2 ist.

2. Verfahren zur Herstellung eines Salzes gemäß dem Anspruch 1, welches die folgenden Schritte umfasst:
- Verestern der zweifach säurefunktionellen Aminosäuren L-Asparaginsäure und/oder L-Glutaminsäure sowie möglicherweise zusätzlich von einfach säurefunktionellen Aminosäuren, die aus Proteinhydrolysaten stammen, mit einem geradkettigen oder verzweigten Alkohol;
- selektive Hydrolyse auf chemischem oder enzymatischem Wege der Esterfunktionen in α-Stellung bei in der Mischung vorhandenen Diestern der Aminosäuren L-Asparaginsäure und L-Glutaminsäure;
- N-Acylierung sämtlicher Aminosäureester in der Mischung, indem ein Säurehalogenid mit einem gesättigten oder ungesättigten Alkyl- oder Arylrest in organischem Milieu einwirken gelassen wird;
- Bildung von Mononatrium- oder Monokaliumsalzen an der freien Carboxylsäurefunktion, die sich in α-Stellung der β-Monoesterderivate der N-Acyl-L-asparaginsäure und der γ-Monoesterderivate der N-Acyl-L-glutaminsäure befinden, indem die entsprechenden Mononatrium- oder Monokaliumsalzen einwirken gelassen werden;
wobei dieses Verfahren durch den folgenden Schritt **gekennzeichnet** ist:
- Ersetzen der basischen Mononatrium- oder Monokaliumsalze der β-Monoester der N-Acyl-L-asparaginsäure und der γ-Monoester der N-Acyl-L-glutaminsäure durch Metallsalze, die sich von zweiwertigen, dreiwertigen, vierwertigen, fünfwertigen, sechswertigen oder siebenwertigen Metallen, Erdalkalimetallen und Übergangsmetallen einschließlich der Lanthanide und der Actinide ableiten.

3. Salze gemäß dem Anspruch 1 oder solche, die durch das Verfahren gemäß dem Anspruch 2 erhalten wurden, **dadurch gekennzeichnet, dass** die komplexbildenden Metalle Mⁿ⁺ aus den folgenden zweiwertigen Kationen (n=2) gewählt sind: Zink, Kupfer, Nickel, Mangan, Magnesium, Calcium, Kobalt, Vanadium, Titan, Chrom, Eisen, Silizium und Cadmium.

4. Salze gemäß dem Anspruch 1 oder solche, die durch das Verfahren gemäß dem Anspruch 2 erhalten wurden, **dadurch gekennzeichnet, dass** die komplexbildenden Metalle Mⁿ⁺ aus den folgenden dreiwertigen Kationen (n=3) gewählt sind: Aluminium, Rhodium, Gallium und Gold.

5. Salze gemäß dem Anspruch 1 oder solche, die durch das Verfahren gemäß dem Anspruch 2 erhalten wurden, **dadurch gekennzeichnet, dass** die komplexbildenden Metalle Mⁿ⁺ aus den folgenden vierwertigen Kationen (n=4) gewählt sind: Selen, Platin und Palladium.

6. Salze gemäß dem Anspruch 1 oder solche, die durch das Verfahren gemäß dem Anspruch 2 erhalten wurden, **dadurch gekennzeichnet, dass** die komplexbildenden Metalle Mⁿ⁺ aus den folgenden fünfwertigen Kationen (n=5) gewählt sind: Tantal und Bismut.

7. Salze gemäß dem Anspruch 1 oder solche, die durch das Verfahren gemäß dem Anspruch 2 erhalten wurden, **dadurch gekennzeichnet, dass** die komplexbildenden Metalle Mⁿ⁺ aus den sechswertigen Kationen (n=6) wie Molybdän oder aus den siebenwertigen Kationen (n=7) wie Rhenium gewählt sind.

8. Verfahren gemäß dem Anspruch 2, **dadurch gekennzeichnet, dass** die zweifach säurefunktionellen Aminosäuren L-Asparaginsäure und/oder L-Glutaminsäure einzeln oder in Mischung oder aber in Kombination mit tierischen oder pflanzlichen Proteinhydrolysaten vorgelegt werden.

9. Verfahren gemäß dem Anspruch 2, **dadurch gekennzeichnet, dass** die Proteinhydrolysate ausgehend von den folgenden Proteinen gewählt sind: Seidenfibroin von *Bombyx mori,* Weizen, Mais, Soja, Sonnenblume, Lupine, Kasein sowie die Meeresalgen *Laminaria digitata, Ascophyllum nodosum, Fucus vesiculosus, Chondrus crispus, Lithothamnium calcareum, Alsidium helminthocorton* und *Digenea simplex*

10. Verfahren gemäß dem Anspruch 2, **dadurch gekennzeichnet, dass** die tierischen oder pflanzlichen Proteinhydrolysate im Vorfeld mit den zweifach säurefunktionellen Aminosäuren L-Asparaginsäure und/oder L-Glutaminsäure derart angereichert werden können, dass deren Gewichtsanteil 5 bis 95 % entsprechen kann, bezogen auf die Proteinhydrolysate.

11. Verfahren gemäß dem Anspruch 2, **dadurch gekennzeichnet, dass** es sich bei den gebildeten Salzen um die zweiwertigen Salze von Zink-di(N-Palmitoyl-L-glutaminsäure-γ-methylester) und um die zweiwertigen Salze von Zink-di(N-Palmitoyl-L-asparaginsäure-β-methylester) handelt.

12. Verfahren gemäß dem Anspruch 2, **dadurch gekennzeichnet, dass** es sich bei den gebildeten Salzen um die zweiwertigen Salze von Kupfer-di(N-Palmitoyl-L-glutaminsäure-γ-methylester) und um die zweiwertigen Salze von Kupfer-di(N-Palmitoyl-L-asparaginsäure-β-methylester) handelt.

13. Verfahren gemäß dem Anspruch 2, **dadurch gekennzeichnet, dass** es sich bei den gebildeten Salzen um die zweiwertigen Salze von Zink-di(N-Palmitoyl-L-glutaminsäure-γ-methylester) mit Seidenfibroinhydrolysaten handelt.

14. Verfahren gemäß dem Anspruch 2, **dadurch gekennzeichnet, dass** es sich bei den Salzen um die dreiwertigen Salze von Aluminium-tri(N-Palmitoyl-L-glutaminsäure-γ-methylester) mit Kuhmilchkasein-Hydrolysaten handelt.

15. Verwendung der Salze gemäß den Ansprüchen 1 oder 3 bis 7 oder aber solchen, die gemäß einem der Ansprüche 2 oder 8 bis 14 erhalten wurden, in pharmazeutischen, kosmetischen oder dermatologischen Zusammensetzungen.

16. Verwendung der Salze gemäß dem Anspruch 15 zur Herstellung von Molekülen mit einer entzündungshemmenden Wirkung.

17. Verwendung der Salze gemäß dem Anspruch 15 zur Herstellung von Molekülen mit einer antimikrobiellen Wirkung.

18. Verwendung der Salze gemäß dem Anspruch 15 zur Herstellung schweißhemmenden Mitteln.

19. Verwendung der Salze gemäß dem Anspruch 15 zur Herstellung von Molekülen, die eine Wirkung auf die Regeneration der Hautzellen ausüben.

20. Verwendung der Salze gemäß dem Anspruch 15 zur Herstellung von Mitteln, die Radikale abfangen.
